# EUROPEAN PATENT APPLICATION

(11) **EP 2 253 707 A2**
(43) Date of publication of application: **24.11.2010**
(21) Application number: 10004421.3
(22) Date of filing: 06.03.2003
(51) Int. Cl.: C12N 15/31, C12N 15/63, C07K 14/245, C07K 16/12, A61K 39/108, G01N 33/53

(54) **Expec-specific proteins, genes encoding them and uses thereof**

(30) Priority: 06.03.2002 EP 02290556
(62) Divisional of application: 03724923.2
(71) Applicant: LABORATOIRE BIODIM, 75007 Paris (FR)
(72) Inventor: Escaich, Sonia, 75012 Paris (FR)
(74) Representative: Peaucelle, Chantal

(57) **Abstract**

The invention relates to isolated antigenic polypeptides obtainable by a process comprising the steps of :
- selecting on the basis of sequence analysis those of the polypeptides which are either located in the outermembrane or secreted by the bacteria,

2- identifying the genes coding for said polypeptides which are conserved in B2/D clinical isolates,
3- purifying the polypeptides identified in step 1, which are found in step 2 to be conserved in B2/D isolates,
4- testing the polypeptides for immunogenicity using animals models.

Application for making vaccine compositions.

## Description

The invention relates to new products specific to pathogenic strains, particularly to extra-intestinal *E. coli* strains.

It more particularly relates as products to antigenic polypeptides and antibodies directed against said polypeptides and to their use as vaccines and in immunotherapy, respectively.

Although *Escherichia coli* is probably the best known bacterial species and is one of the most common isolated in clinical microbiology laboratories, misconceptions abound regarding the various types of *E. coli* and the infections they cause.

*E. coli* strains of biological significance to humans can be broadly classified in 3 major groups:
1. Commensal strains, which are part of the normal flora.
2. Intestinal pathogenic strains, which are not part of the normal flora. This group contains various pathotypes (EPEC, EHEC, ETEC, EIEC) not including *Shigella*.
3. Extra-intestinal strains (ExPEC) which are responsible for infections outside the gastro-intestinal (GI) tract, but can also be part of the normal flora. All hosts, either immunocompromised or not are susceptible to these infections.

ExPEC strains are responsible for the majority of the urinary tract infections (UTI) particularly cystitis, pyelonephritis, and cathether associated infections.

They are also responsible for abdominal infections, nosocomial pneumoniae, neonatal meningitidis, soft tissue infections, and bone infections. Each one of these localizations can lead to bacteremia with a risk of sepsis in case of organ failure. ExPEC strains are indeed the most common Gram negative bacilli isolated from blood cultures.

750 000 cases of bacterial sepsis occur each year in the US, and are responsible for 225 000 deaths. In a recent study on 1690 cases of sepsis, it was shown that the main bacteria species identified is ExPEC (16% of the cases) and then *S.aureus* (14% of the cases) .

These numbers demonstrate the importance of ExPEC strains in both hospital and community acquired infections.

ExPEC strains correspond to a homogenous subset of *E. coli* strains. Analysis of phylogenetic relationships among *E. coli* strains by MLEE has revealed that *E. coli* belong to 4 main phylogenetic groups designated A, B1, B2 and D.

The pathogenesis of ExPEC strains is that of extra-cellular microorganisms, i.e., they are well adapted to growth in the extra-cellular fluids and efficiently resist phagocytosis by polymorphonuclear. Initial studies have shown that virulence factors known to be important for the extra-cellular growth are mainly found in B2/D *E. coli.,* thus suggesting that B2/D subgroups contain most of the ExPEC strains. This was reinforced by experiments performed on animals showing that B2/D strains are more virulent than A and B1 strains. Subsequent epidemiological studies have indeed confirmed these hypotheses. B2/D isolates are those predominantly responsible for neonatal meningitidis (87%) and community or nosocomial acquired urosepsis, (93 % and 85%, respectively).

Similar results have been reported for cystitis (70% are due to the sole B2 *E. coli*) thus demonstrating that the importance of ExPEC strains.

These recent findings demonstrate that the B2/D subgroup of strains is the *E. coli* core genome the best adapted to growth in extra-cellular fluids.

In addition to this core genome, ExPEC strains have various pathogenicity islands which encode virulence factors associated with the different pathogenesis of extra-intestinal *E. coli* infections (UTI, urosepsis, neonatal meningitidis...). Among the main virulence factors are the capsule, which is well-known to be important for extra-cellular growth, and the iron chelation systems (aerobactin and enterochelin, for example). In addition, depending on the pathogenesis, these strains can produce toxins (CNF, hemolysin...), adhesins (pap, sfa...) and other iron chelation systems.

The notion that B2/D *E. coli* correspond to a distinct subset of pathogenic *E. coli* strains is reinforced by the fact that B2/D *E. coli* are not broadly isolated from the stools of humans. They were recovered from only 11% of individuals, whereas A and B1 subgroups are present in the stools of 74% of the individuals of a human population.

As mentioned above the pathogenesis of ExPEC strains relies on their ability to multiply in the extra-cellular fluids and to resist bactericidal activity of the complement and phagocytosis by polymorphonuclear. Therefore, as for other extra-cellular pathogens (*Haemophilus influenzae, Streptococcus pneumonieae* and *Neisseria meningitidis*) a protective antigen against ExPEC has to induce antibodies that promote opsonisation and/or the bactericidal activity of serum.

Considering the above statements, an efficient antigen has to be largely represented among the population of B2/D *E. coli.* Similarly to other extra-cellular pathogens, the capsular polysaccharide would be an ideal antigen, however most pathogenic B2 strains express the K1 polysaccharide. The latter has a structure identical to that of group B meningococcus, which is non-immunogenic and shares common antigens with the brain. Another possible target may be the lipopolysaccharide (LPS). However there are a large number of different LPS serotypes that are shared by various subgroups.

The inventors have now found that some specific components coded by the B2/D genome, but absent from A and B1 *E. coli* strains, are particularly useful as antigens and can specifically prevent the pathologies due to ExPEC strains. Homologs of these antigenic components can be found in other pathogenic bacterial species and therefore are useful to prevent the pathologies caused by these bacteria. Accordingly, any reference to products specific to ExPEC strains and to their uses will encompass components in these species.

For example homologuous antigens could be present in the following species and be as such used for prevention of disease due to the bacteria:

*Pseudomonas aexuginosa, Escherichia coli* O157:H7,*Yersinia pestis, Vibrio cholerae, Legionella pneumophila, Salmonella enterica, Salmonella typhimurium, Haemophilus influenzae, Neisseria meningitidis, Neisseris gonorrhoeae, Bacillus anthracis, Burkholderia cepacia, Campylobacter jejuni, Chlamydia pneumoniae, Chlamydia trachomatis, Clostridium botulinum, Clostridium difficile, Cryptococcus neoformans, Enterobacter cloacae, Enterococcus faecalis, Helicobacter pylori, Klebsiella pneumoniae, Mycobacterium leprae, Mycobacterium tuberculosis, Pseudomonas aeruginosa, Salmonella paratyphi, Salmonella typhi, Staphylococcus aureus, Klebsislla pneumoniae, Listeria monocytogenes, Moxarella catarrhalis, Shigella dysenteriae, Shigella flexneri, Shigella sonnei, Staphylococcus epidermidis, Streptococcus pneumoniae,* and any species falling within the genera of any of the above specie.

It is then an object of the invention to provide new isolated antigenic polypeptides, and polynucleotides belonging to the core B2/D genome and not present in commensal *E, coli.*

Another object of the invention is to provide antibodies raised against such antigenic polypeptides, or peptidic fragments.

It is still another object of the invention to provide vectors and host cells containing said polynucleotides.

Another object of the invention is to provide vaccine compositions specific to extra intestinal infections caused by ExPEC and pathologies caused by other pathogenic strains expressing antigenic polypeptides homologous to the ExPEC antigenic polypeptides.

The invention also relates to means for detecting and treating a development of *E. coli* in a human or animal compartment which is extra-intestinal (systemic and non-diarrhoeal infections, such as septicaemia, pyelonephritis, or meningitis in the newborn).

The isolated antigenic polypeptides used according to the invention are selected among polypeptides specific to B2/D *E. coli* strains and not present in A and B1 isolates of *E. coli*. They are encoded by genes belonging to the core B2/D genome and are not present in commensal *E. coli*.

They have a sequence selected in the group comprising the sequences of SEQ ID N°11 to N°66 or 133-145 or homologous sequences with a minimum of 25% of identity with the whole sequences SEQ ID N°17 to N°66, or 133-145, respectively.

The isolated polypeptides having SEQ ID N° 14, 15, 17, 21, 22, 23, 28, 29, 30, 32, 36, 38, 39, 41-44, 45, 49, 50, 52 to 55, 58, 60, 63, 133-138 are new polypeptides and therefore are part of the invention.

The invention also relates to homologous isolated antigenic peptides, comprising polypeptides having at least 25% identity to a polypeptide having a sequence SEQ ID N° as above defined, more particularly having SEQ ID N°14, 15, 17, 21, 22, 23, 28, 29, 30, 32, 36, 38, 39, 41-44, 46, 49, 50, 52 to 55, 58, 60, 63, 133-138, or at least 25% identity to a fragment comprising at least 5, at least 10, at least 20, at least 30, at least 40, at least 50, at least 60 or more than 60 consecutive amino acids of a polypeptide having a sequence corresponding to said SEQ ID N°a, as determined using BLASTP or BLASTX with the default parameters.

Said polypeptides are obtainable by a process comprising the steps of :
a- selecting on the basis of sequence analysis those of the polypeptides which are either located in the outermembrane or secreted by the bacteria,
b- identifying the genes coding for said polypeptides which are conserved in B2/D clinical isolates,
c- purifying the polypeptides identified in step a, which are found in step 2 to be conserved in the B2/D isolates,
d- testing the polypeptides for immunogenicity using animals models.

By the term "conserved", it is meant, according to the invention, that the genes coding for the polypeptides are present with a frequency of at least 50% in B2/D isolates, preferably greater than 60%, more preferably greater than 80% and even more preferably greater than 85%, and in less than 40% in A/B isolates, preferably in less than 20%, more preferably in less than 15%.

The animal models used in step c are infected adult animals, eventually immunodepressed.

The adult animals particularly mice, are infected intraperitoneally, the endpoint being the animal death and/or bacteremia measurement.

The animals can be immunodepressed by injection, for example, of cyclophosphamide which induces a neutropenia. Such a model will validate the use of the antigen for prevention of *E. coli* sepsis in immunodepressed patients. Another animal model could be for example 2 to 3 day old infant mice.

The variants or fractionnal sequences conserving the B2/D properties and which are antigenic as defined in step 4 of the above process are also part of the invention. The term "variant" is herein intended to mean any sequence having insertions and/or deletions and/or substitutions with respect to the parent sequence. The term "fractional" is herein intended to mean any fragment of the parent sequence.

The invention also relates to the use of isolated polynucleotides coding for a polypeptide such as above defined according to the universal genetic code and taking into account the degeneracy of this code. The term "polynueleotide" encompasses any nucleotidic sequence such as DNA, including cDNA, RNA, including mRNA.

Said polynucleotides have preferably sequences corresponding to SBQ ID N°77 to SEQ ID N°132 or 146 to 158 .

More preferably, said polynucleotides have sequences corresponding to SEQ ID N° 80, 81, 83, 87, 88, 89, 94, 95, 96, 98, 102, 104, 105, 107-110, 112, 115, 116, 118, 119, 125, 127, 130, 132, 135, 146-151.

The invention also relates to the homologs to said polynuoleotides. Said homologs may have at least 25% identity to a polynueleotide having said sequences, or at least 25% identity to a fragment comprising at least 15, at least 30, at least 60, at least 90, at least 120, at least 150, at least 180 or more than 180 consecutive nucleotide of a polynucleotide having one of said SEQ ID N°s, as determined using BLASTN with the default parameters, and are encompassed by the invention inasmuch as they are capable of coding for a polypeptide having the antigenic properties of those according to the invention.

The present application is also aimed towards any vector comprising at least one of said polynucleotidss and also any cell transformed by genetic engineering, **characterized in that** it comprises, by transfection, at least one of said polynucleotide and/or at least one vector according to the invention, and/or in that said transformation induces the production by this cell of at least one polypeptide corresponding to a polynucleotide such as above-defined.

The invention also relates to a process for isolating and identifying antigenic polypeptides, therefore useful as vaccine for *E. coli.*

Such a process comprises the steps of
a- selecting on the basis of sequence analysis those of the polypeptides which are either located in the outermembrane or secreted by the bacterial,
b- identifying the genes coding for said polypeptides which are conserved in B2/D clinical isolates,
c- purifying the polypeptides identified in step a, which are found in step 2 to be conserved in B2/D isolates,
d- testing the polypeptides for immunogenicity using animals models.
   The selected antigenic polypeptides, alone or in combination, are capable of inducing an antibody response for prevention of infections due to ExPEC strains regardless of the pathogenesis and of the infection site (UTI, pyelonephritis, sepsis, bacteremia, neonatal meningitis).

Such polypeptides particularly have sequences SEQ ID N°1 to SEQ ID N°66, or 133-145 or correspond to homologous sequences.

The invention thus relates to vaccine compositions specific to *E. coli* extra-intestinal infections, comprising an effective amount of at least one antigenic polypeptide or fragment thereof as above defined, with a carrier, particularly at least one polypeptide of SEQ ID N°1 to SEQ ID N°66; except SEQ ID N°8, and 133-145 and the homologous polypeptides.

Such vaccine compositions are particularly useful for preventing urinary system infections, pyelonephritis, sepsis, bacteremia, neonatal meningitis.

The vaccine compositions of the invention are indicated for :
- immunodepressed patients, ideally before the start of the immunosuppressive therapy : patients suffering from cancer, diabetes, leukaemia, transplant patients, patients receiving long-term steroids therapy.
- Patients before surgery where there is a high risk of *E. coli* infections (abdominal surgery) .
- In all these cases, the *E. coli* vaccine of the invention could be administered in association with a *Staphylococcus* aureus vaccine,
- Patients with recurrent UTI, especially after one episode of pyelonephritis.
- The prevention of neonatal infections will require vaccination of the mother, implying vaccination long before pregnancy to avoid potential problem. Ideally such a vaccine should be associated with a Group B *Streptococcus* polysaccharide vaccine in order to also prevent late onset neonatal infections. It should be pointed out that the induction of a level of antibodies against B2/D *E. coli* in pregnant women would also prevent UTI, which are always a risk in the context of a pregnancy.

The formulation and the dose of said vaccine compositions can be developped and adjusted by those skilled in the art as a function of the indication targeted, of the method of administration desired, and of the patient under consideration (age, weight).

These compositions comprise one or more physiologically inert vehicles, and in particular any excipient suitable for the formulation and/or for the method of administration desired.

For example the vaccine could be a suspension of the purified polypeptide in sterile water with aluminium based mineral salt as adjuvant and be administred subcutanously with a first and boosting injection.

The antibodies raised against the above-identified polypeptides are also part of the invention.

They are capable of binding to said polypeptides in physiological-type conditions (*in vivo* or mimicking *in vivo)* when administered to a human or animal organism, and ELISA-type conditions when said binding product is intended to be used in assays and methods *in vitro.* Such antibodies advantageously inhibit the extra-intestinal growth of ExPEX strains in human or animal.

They are particularly useful for immunotherapy applications with antibodies specific to polypeptidic antigens, for treatment and prevention of severe infections in at risk populations such as neonates or patients undergoing surgical procedures. For these applications specific human monoclonal antibody (Mab) will be derived from the peptides or polypeptides.

The methods for manufacturing such antibodies using the polypeptides according to the invention are available to those skilled in the art. They are conventional methods which comprise, in particular, the immunization of animals such as rabbits and the harvesting of the serum produced, followed optionally by the purification of the serum obtained. A technique suitable for the production of monoclonal antibodies is that of Köhler and Milstein (Nature 1975, 256:495-497).

Said antibodies do not recognize the cells of the human or animal to which it is intended.

In particular for immunotherapy applications with monoclonal antibodies specific to polypeptidic antigens, for treatment and prevention of severe infections in at risk populations such as neonates or patients undergoing surgical procedures. For these applications specific human monoclonal antibody will be derived from the peptides or polypeptides.

The antibodies or fragments thereof are advantageoulsy humanized when intended for a human administration.

Alternatively, humanized Mab could be derived from murine or rat Mab specific of the antigen. These fully humanized Mab are constructed using conventional molecular techniques to graft complementarity-determining regions from the parent murine or rat antibacterial antibody into human IgG1 kappa heavy and light-chain frameworks.

The present invention is also aimed towards the use, in an effective amount, of at least one of polypeptides having SEQ ID N°14, 15, 17, 21, 22, 23, 28, 29, 30, 32, 36, 38, 39, 41-44, 46, 49, 50, 52 to 55, 58, 60, 63, 133-138, antibodies or polynucleotides for the diagnosis of the presence or absence of undesirable extra-intestinal *E. coli*, and/or for the diagnosis of an extra-intestinal *E. coli* infection.

The detection of the presence or absence of such compounds can in particular be carried out by nucleotide hybridization, by *PCR* amplification or by detection of their polypeptide products. Detection of the presence of such compounds makes it possible to conclude that a B2/D *E. coli* strain is present.

The invention also relates to pharmaceutical compositions for alleviating and/or preventing and/or treating an undesirable growth of *E. coli* comprising an effective amount of at least one polypeptide as above defined, particularly having SEQ ID N°1-66 to 133-145, in combination with a pharmaceutically acceptable carrier.

Preferred pharmaceutical compositions comprise at least one polypeptide having SEQ ID N°14, 15, 17, 21, 22, 23, 28, 29, 30, 32, 36, 38, 39, 41-44, 46, 49, 50, 52 to 55, 58, 60, 63, 133-138,

The present application is also aimed towards any use of a polypeptide such as above defined for the manufacture of a composition, in particular of a pharmaceutical composition, intended to alleviate and/or to prevent and/or to treat an undesirable growth of *E. coli,* such as an *E. coli* infection, (for example systemic and non-diarrhoeal infections), the presence of extra-intestinal *E. coli* or a sanitary contamination.

The present invention is illustrated by the examples which follow and which are given in a non limiting capacity and with reference to figures 1 and 2, wherein
- Figure 1 represents a protein purification result after cloning and expression, and
- Figure 2 is a picture of the DNA array after hybridization with the genomic DNA from a B2/D reference strain.

### Example 1: Assay for the immunogenicity of a selected polypeptide from sequences 1-66 and 133-145 (except SEQ ID N°8)

### Cloning expression and purification of the selected polypeptide.

The nucleic acid having SEQ ID N°95 encoding the polypeptide corresponding to SEQ ID N°28 was cloned without the signal sequence (coding the 16 first amino acids) in a prokaryotic expression vector according to classical methods for cloning. The recombinant plasmid was used to transform the *E. coli* strain BL21. Transformed cells containing the recombinant plasmid were selected in LB medium with 100µs/ml ampicillin. Individual clones are picked and grown in preaence of IPTG 1mM to induce recombinant protein expression. Total protein content of the culture cells was extracted by cell lysis. Recombinant protein was purified by affinity columns.

### Protein purification after cloning and expression

Total cell lysat of IPTG-induced bacteria were mixed with Ni-NTA matrix (Qiagen^{®}) for 60 min et 4°C and loaded into a column. After washing the column to remove non specific binding, the recombinant protein was eluate 3 times with 1 ml elution buffer pH 5.9. The protein was then eluate 4 times with 1 my elution buffer pH 4.5.

Figure 1 represents a Coomassie blue stained SDS gel of recombinant protein after affinity column purification: PM: markers E1-4: sample collected from each purification fraction. Arrow indicate the band corresponding to the recombinant protein.

### Test for immunogenicity in an animal model

Polypeptide preparation from SEQ ID N°28 was injected to Swiss mice to induce an antibody response as follows :

At d0 a first immunisation was done by injecting 20µg of the protein at in 100µg solution of PBS and complet Freund adjuvant (1:1). Control animals were injected with 100µl solution of PBS and complet Freund adjuvant (1:1).

Boosting injection at d21 with 10µg of protein in 100µl PBS and complet Freund adjuvant (1:1).

Sera from vaccinated animals was prepared from blood drawn by puncture in the tail of the mice.

Detection of specific antibodies in animal sera, at d20 before the boosting injection, was performed by western blot according to standart protocol. Purified polypeptide was subjected to electrophoresis (10µg per lane) and transfert to nitrocellulose membrane.

The membranes were then saturated by incubation 35 min with PBS/Tween20 0.1%/powder milk 5%.

Diluted sera was incubated with the membrane for 45 min. Membranes were washed three time 5 min with PBS/tween. Bound antibodies were then recognized by an anti-mouse IgG coupled to horseradish peroxidase enzyme. After washing 3 times with PBS/Tween and 3 time with PBS, enzymatic activity was revealed by addition of chromogenic substrate DAB and hydrogen peroxyde.

Results ; Sera from vaccinated animal, diluted at 1/100 revealed a unique band corresponding to the injected polypeptide. No antibody to the polypeptide could be detected in sera from control animals.

At d42, 300 µl of cyclophosphamide and 200µl at d45 were injected IP in the mice to induce neutropenia in order to increase the susceptibility to the challenge infection.
At d46 vaccinated and control mice were challenged by intraperitoneal injection of the wt B2/D strain C5 of *E. coli* at a dose equal to 10 time the LD50 (letal dose).

Immunogenicity of the selected polypeptide and protection conferred by vaccination with the seleted polypeptide was assessed by the survival of vaccinated animals three days post challenge.

Another example of vaccination to demonstrate immunogenicity of polypeptide:
- 24 Balb/c mice, female, 6 weeks old were immunized by subcutaneous Injection of the protein in Complete Freund's adjuvant, and 14 control mice were injected with CFA and PBS
- 3 weeks later a boost injection of 10 µg of protein in with incomplete Freund's adjuvant.

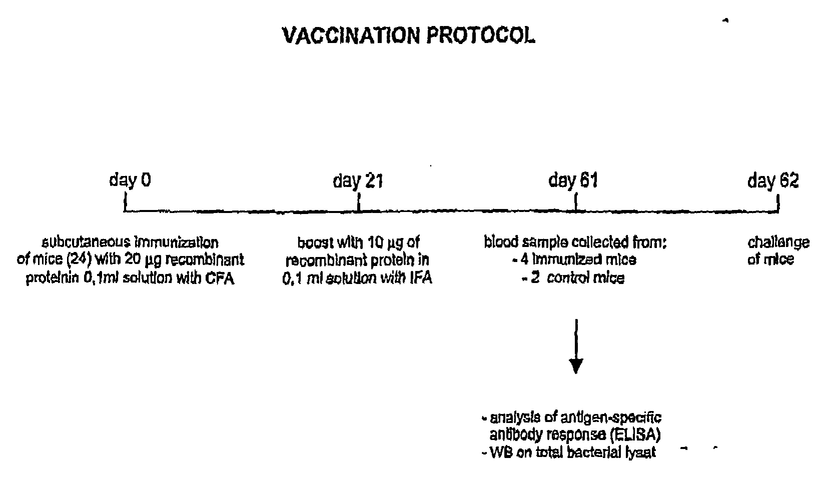

Before challenge at d62, sera was collected at d61 to analyze the antibody response in the vaccinated animals.
• WB analysis of sera from immunized mice were performed to detect the antibody response to the recombinant proteine used for immunization as described above,
• An ELISA assay was set up to measure antibody titer in vaccinated animals:
- Each recombinant protein was coated on a 96-well plates with (200 ng/100 µl coating buffer), plates are saturat with 3%BSA in PBS.
- Twofold serial dilution of sera were made in PBS 1X/1% BSA from 1:500, to 1: 1024000 and incubated on the plate, antiboby binding was reavealed using a rabbit Anti-mouse IgG conjugated with Peroxydase and OPD(o-phenylenediamine) chromogen substrate.
- Read the OD₄₉₅ with Sanofi Diagnostics Pasteur FR2100^{®}
• Results:

**Table 1. Sera titer against recombinant protein by ELISA**

| **SEQ ID** | **serum1** | **serum 2** | **serum 3** | **serum 4** | **Control** | **recombinant protein** |
|---|---|---|---|---|---|---|
| **2** | **128** | **64** | **128** | **512** | **<0,5** | |
| **140** | **>64** | **>64** | **>64** | **>64** | **<0,5** | **+** |
| **31** | **>64** | **>64** | **>64** | **>64** | **<0,5** | **+** |
| **49** | **>64** | **>64** | **>64** | **>64** | **<0,5** | **+** |
| **51** | **>64** | **>64** | **>64** | **>64** | **<0,5** | **+** |
| **25** | **>64** | **>64** | **>64** | **>64** | **<0,5** | **+** |
| **7** | **16** | **8** | **>64** | **<0,5** | **<0,5** | **+** |
| **19** | **>64** | **>64** | **>64** | **>64** | **0,5** | **+** |
| **3** | **>64** | **>64** | **>64** | **>64** | **<0,5** | **+** |
| **26** | **512** | **128** | **64** | **256** | **<0,5** | **+** |
| **18** | **>64** | **>64** | **>64** | **>64** | **0,5** | **+** |
| **32** | **>64** | **>64** | **>64** | **>64** | **0,5** | **+** |
| **53** | **>64** | **>64** | **>64** | **>64** | **0,5** | **+** |
| **587** | **>64** | **16** | **32** | **32** | **<0,5** | **+** |
| **11** | **>64** | **32** | **32** | **64** | **<0,5** | **+** |
| **36** | **512** | **256** | **512** | **256** | **<0,5** | **+** |
| **10** | **32** | **128** | **256** | **128** | **0,5** | **+** |
| **47** | **512** | **512** | **512** | **512** | **0,5** | **+** |
| **20** | **1024** | **256** | **256** | **512** | **<0,5** | **+** |
| **17** | **1024** | **512** | **128** | **512** | **<0,5** | **+** |

• To assess the ability of sera to recognize the native antigen expressed by the bacteria, western blot was also performed on whole bacteria lysat.
To this end, bacteria were grown in LB medium supplemented or not with iron chelator until OD600=0.5-0.6 and pelleted by centrifugation 5 minutes at 10000 rpm. The pellet was lysed by resuspension in 1X loading buffer containing SDS and heated 5 min at 95°C before migration on the gel.Western blot assay was then performed with sera from controls and vaccinated animals.
Results in table 2 shows the results obtained with Sera from vaccinated mice against recombinant protein and against *E.coli* lysat.

**Table 2: reactivity in Western Blot of sera from mice vaccinated with polypeptides encoded by the different ORFs**

| **SEQ IN N°** | **whole cell lysats** | **recombinant protein** |
|---|---|---|
| **2** | **+** | **+** |
| **140** | **+** | **+** |
| **26** | **+** | **+** |

• Protection assay, end point: mortality
At d62, 20 vaccinated and 10 control mice were challenged with an *E.coli* virulent strain belonging to B2 group at a dose equal to the LD 50 (5.10⁵ cfu/mice) by intraperitoneal injection. Mortality is recorded at 48h, results in Table 3 are expressed as a percentage of protection representing the difference of survival in vaccinated versus control mice goups.

**Table 3 : Protection obtained in mice challenged after inmunization with proteines encoded by the corresponding ORFs.**

| **SEQ ID N°** | **% protection** |
|---|---|
| **2** | **52** |
| **26** | **66** |
| **36** | **46** |
| **10** | **30** |
| **47** | **60** |
| **20** | **25** |

• Protection assay, end point: bacteremia
At d62, 10 vaccinated and 5 control mice were challenged with an Ecoli virulent strain belonging to B2 group at a dose equal to the 1/5 of the LD 50 (1.10⁵ cfu/mice) by intraperitoneal injection.With this infectious dose the mice survived the infection at d48. At 48h blood was collected for each mice in presence of heparin .To assess bacteremia , the blood was plated on LB media and colonies count measured after overnight culture.

### Example 2: Distribution of the DNA sequence of ORFs specific for B2/D group of E.coli in clinical isolates.

To make a DNA arrays membrane specific for B2/D group of *E.coli ,* DNA corresponding to ORFs that were identified as specific for B2/D core genome of *E.coli* was amplified by PCR and spotted on nylon membrane using standard methods to those skilled in the art.

Chromosomal DNA from 30 *E.coli* clinical isolate strains ( of which 23 were from pathological conditions and 6 isolated from human normal flora), was prepared and radiolabelled with 33P.

DNA from these clinical isolates was then hybridized to the B2/D specific DNA array, the results were read by a phosphoimager and spots reactivity was analyzed with an image analysis software. If hybridization gave a positive signal for a particular ORF, this ORf is considered to be present in the genome of the isolate. Quality control of the array is the hybridization of a probe DNA from a reference strain of *E.coli* as shown in figure 2, which represents a picture of the DNA array after hybridization with the genomic DNA from a B2/D reference strain.

The details of the method used for these experiments has been described previously in Tinsley et al. Methods Enzymol. 2002, 358; 188-207.

Results presented in Table 4 are expressed as the frequency of each ORF detection in the three different group A, B and D of *E.coli* strains.

**Table 4 :Presence of Orfs encoding antigens in E.coli clinical isolate genomes**

| | Clinical isolate groups | | |
|---|---|---|---|
| Frequency of positive signal % | A | D | B2 |
| SEQ ID N° | N=6 | n=5 | n=18 |
| 86 | 17 | 20 | 100 |
| 119 | 0 | 80 | 100 |
| 137 | 0 | 0 | 100 |
| 77 | 0 | 100 | 94 |
| 78 | 0 | 100 | 100 |
| 79 | 0 | 100 | 100 |
| 80 | 0 | 0 | 100 |
| 84 | 0 | 0 | 100 |
| 82 | 0 | 0 | 100 |
| 88 | 0 | 0 | 100 |
| 83 | 0 | 0 | 94 |
| 85 | 0 | 0 | 78 |
| 88 | 0 | 0 | 56 |
| 81 | 33 | 20 | 100 |
| 89 | 33 | 60 | 67 |
| 90 | 0 | 0 | 61 |
| 91 | 17 | 80 | 67 |
| 94 | 0 | 0 | 100 |
| 92 | 0 | 0 | 100 |
| 93 | 0 | 0 | 100 |
| 96 | 0 | 100 | 100 |
| 85 | 17 | 100 | 100 |
| 97 | 0 | 70 | 100 |
| 98 | 0 | 0 | 100 |
| 99 | 0 | 0 | 78 |
| 101 | 0 | 80 | 100 |
| 102 | 0 | 0 | 100 |
| 104 | 0 | 0 | 11 |
| 103 | 0 | 0 | 100 |
| 105 | 0 | 0 | 67 |
| 100 | 0 | 100 | 100 |
| 111 | 17 | 0 | 61 |
| 115 | 0 | 0 | 78 |
| 114 | 0 | 0 | 83 |
| 113 | 0 | 0 | 94 |
| 120 | 33 | 80 | 16 |
| 125 | 17 | 0 | 89 |
| 109 | 0 | 0 | 33 |
| 110 | 0 | 0 | 17 |
| 124 | 0 | 0 | 72 |
| 126 | 0 | 80 | 78 |
| 116 | 0 | 0 | 33 |
| 112 | 0 | 0 | 22 |
| 106 | 17 | 0 | 33 |
| 117 | 33 | 80 | 11 |
| 132 | 33 | 80 | 22 |
| 122 | 0 | 0 | 22 |
| 74 | 0 | 0 | B9 |
| 70 | 0 | 0 | 33 |
| 73 | 0 | 0 | 89 |
| 71 | 50 | 100 | 22 |
| 75 | 0 | 0 | 0 |
| 76 | 0 | 0 | 56 |
| 67 | 0 | 0 | 50 |
| 69 | 100 | 100 | 100 |
| 68 | 67 | 100 | 100 |
| 152 | 0 | 0 | 94 |
| 153 | 0 | 100 | 100 |
| 150 | 0 | 0 | 11 |
| 142 | 83 | 100 | 78 |
| 157 | 67 | 0 | 94 |
| 156 | 17 | 100 | 100 |
| 100 | 0 | 100 | 100 |
| 154 | 0 | 80 | 67 |
| 147 | 0 | 0 | 100 |
| 146 | 17 | 100 | 100 |
| 158 | 17 | 100 | 89 |
| 107 | 17 | 0 | 78 |
| 72 | 50 | 100 | 44 |
| 151 | 0 | 0 | 11 |
| 149 | 0 | 0 | 28 |
| 148 | 0 | 0 | 6 |

### Example 3 : Vaccines compositions intended for prevention of any form of infection by ExPEC.

The polypeptide coded by a sequence comprising SEQ ID N°28 is conjugated with a toxin and added to a physiologically inert vehicle.

This conjugated peptide is optionnally added to a childhood vaccine.

The composition is sterilized and can be injected parenterally, subcutaneously or intramuscularly.

Said composition can also be sprayed onto mucosa with the aid of a spray.

## Claims

1. The isolated polypeptides having SEQ ID N° 14, 15, 17, 21, 22, 23, 28, 29, 30, 32, 36, 38, 39, 41-44, 46, 49, 50, 52 to 55, 58, 60, 63, 133-138.

2. Isolated antigenic polypeptides according to claim 1 obtainable by a process comprising the steps of :
a- selecting on the basis of sequence analysis those of the polypeptides which are either located in the outermembrane or secreted by the bacteria,
b- identifying the genes coding for said polypeptides which are conserved in B2/D clinical isolates,
c- purifying the polypeptides identified in step a, which are found in step b to be conserved in B2/D isolates,
d- testing the polypeptides for immunogenicity using animals models.

3. Isolated polynucleotides, coding for a polypeptide according to claim 1 or 2, according to the universal genetic code.

4. Isolated polynucleotides according to claim 3, having sequences selected in the group comprising SEQ TD N° 80, 81, 83, 87-89, 94-96, 98, 102, 104, 105, 107-110, 112, 115, 116, 118, 119, 126, 127, 130, 132, 135, 146-151.

5. An expression vector comprising at least an isolated polynucleotide according to claim 3 or 4.

6. A host cell comprising an expression vector according to claim 5.

7. A process for isolating and identifying antigenic polypeptides, useful as vaccines comprising the steps of :
a- selecting on the basis of sequence analysis those of the polypeptides which are either located in the outermembrane or secreted by the bacteria,
b- identifying the genes coding for said polypeptides which are conserved in B2/D clinical isolates,
c- purifying the polypeptides identified in step a, which are found in step b to be conserved in B2/D isolates,
d- testing the polypeptides fox immunogenicity using animals models.

8. The process of claim 7, comprising the use of infected adult animals, eventually immunodepressed, and of infant animals as models for neonatal infections.

9. The use of at least one polypeptide or fragment of these polypeptides selected in the group comprising SEQ ID N°1 to SEQ ID N°66(except SEQ ID N°8), or 133-145 as antigens and the homologous sequences.

10. A vaccine composition specific to *E*. *coli* extra-intestinal infections, comprising an effective amount of at least one antigenic polypeptide such as selected by the process of claim 7, or according to claim 9, alone or in combination, particularly at least one polypeptide having a sequence selected in the group comprising SEQ ID N°1 to SEQ ID N°66, or 133-145, except SEQ ID N°8 and the homologous sequences, with a carried.

11. The vaccine composition of claim 10 for preventing urinary system infections, pyelonephritis, sepsis, bacteremia, neonatal meningitidis.
